# DEMANDE DE BREVET EUROPEEN

(11) **EP 0 653 219 A1**
(43) Date de publication de la demande: **17.05.1995**
(21) Numéro de dépôt: 93810794.3
(22) Date de dépôt: 16.11.1993
(51) Int. Cl.: A61M 16/22, B01D 53/04, A61L 9/22, A61M 15/02

(54) **Procédé pour produire un mélange gazeux hypoxique**

(71) Demandeur: TRADOTEC S.A., CH-1214 Vernier (CH)
(72) Inventeur: Tkatchouk, Elena N., RU-103473 Moscow (RU); Staebler, Regula, CH-1214 Vernier (CH)
(74) Mandataire: Charbonnier, Georges R.

(57) **Abrégé**

L'appareil pour effectuer des traitements hypoxique mettant en oeuvre le procédé comprend, disposés en circuit fermé, un masque (1), une valve (2), un robinet à trois voies (4), un filtre (5), un ventilateur (6), un électro-filtre (8), une sonde (12), un analyseur de gaz (13), une valve (2'), et un réservoir (15).

## Description

La présente invention concerne la médecine en général et les traitements hypoxiques en particulier.

Elle a pour objet un procédé pour produire un mélange gazeux composé d'oxygène et d'azote dans les proportions respectives de 8 à 18% et 92 à 82% en volume et un appareil pour des traitements hypoxiques mettant en oeuvre ce procédé.

On connait déjà un procédé pour la production d'un mélange gazeux hypoxique dans lequel on appauvrit en oxygène de l'air en le faisant traverser une membrane en fibres polymériques.

Dans un appareil connu destiné à des traitements hypoxiques qui met en oeuvre ce procédé, le mélange gazeux obtenu est amené dans un espace clos où il est inhalé par le patient.

Cet appareil présente l'inconvénient d'être lourd et encombrant. De plus il doit être installé dans un local spécial ce qui complique son usage pratique.

On connait un autre procédé pour la production d'un mélange gazeux hypoxique dans lequel on comprime de l'air entre deux et dix bar, puis fait passer cet air comprimé à travers une membrane où il s'appauvrit en oxygène ( demande de brevet européen No 042 799 ).

On connait également un appareil mettant ce procédé en oeuvre pour des traitements hypoxiques comprenant un compresseur à membranes, une membrane constituée par des fibres de 2-méthyl pentene-polymérique, un filtre de Petrianov fomé d'une toile composée de différents matériaux en fibres polymériques, un analyseur de gaz et un masque.

Les principaux inconvénients de cet appareil résident dans le fait qu'il nécessite un compresseur et des éléments compliqués d'un coùt relativement élevé.

Le procédé selon la présente invention permet la production d'un mélange gazeux d'une grande pureté, pratiquement stérile, possédant les qualités et remplissant les conditions requises pour des traitements hypoxiques de haute exigence, Ce procédé consiste à appauvrir l'air en oxygène par la méthode de la respiration périodique, méthode basée sur le fait qu'une personne respirant normalement, inspire de l'air et expire un mélange gazeux hypoxique, et à éliminer le dioxyde de carbone, l'humidité et les impuretés du mélange.

Dans ce procédé l'air n'est pas comprimé et le mélange gazeux est à la pression atmosphérique.

L'appareil selon l'invention comprend un masque destiné à être placé sur le visage du patient, au moins une valve et un ou plusieurs filtres destinés à débarrasser le mélange gazeux expiré par le patient, du dioxyde de carbone et de ses impuretés.

L'avantage de cet appareil est de ne pas comporter de compresseur et de pouvoir ainsi le réaliser avec un poids et un volume réduits par rapport aux appareils connus, et partant de pouvoir le transporter facilement d'un lieu à un autre. En outre le mélange gazeux obtenu est d'une grande pureté et pratiquement stérile, conditions à remplir impérativement pour son emploi lors de traitements hypoxiques.

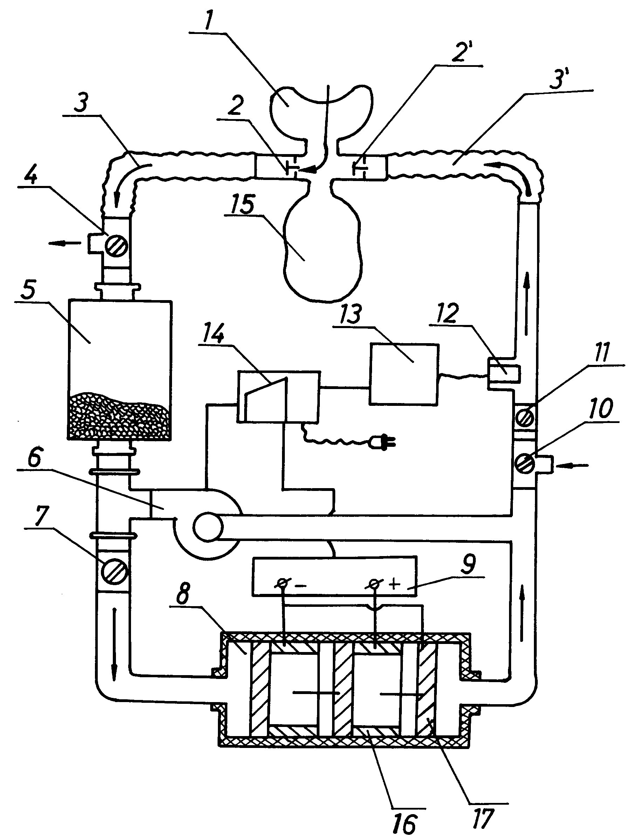

La figure unique du dessin annexé représente , schématiquement et à titre d'exemple non limitatif, une forme d'exécution de l'appareil selon l'invention.

L'appareil représenté comprend, disposés en circuit fermé, un masque 1 destiné à être appliqué sur le visage du patient, deux valves indépendantes 2 et 2', deux tuyaux souples 3 et 3', un premier robinet à trois voies 4, un filtre 5, un ventilateur 6, un robinet 7, un électro-filtre 8, une source de courant électrique continu 9, un second robinet à trois voies 10, un second robinet 11, une sonde d'oxygène 12, un analyseur de gaz 13, un transformateur-redresseur 14, et un réservoir 15.

L'appareil se branche sur le réseau électrique domestique par l'entremise du transformateur-redresseur 14 qui alimente le ventilateur 6, la source 9 et l'analyseur 13.

L'appareil fonctionne comme suit :

### Phase 1

Le patient inspire sans le masque 1 de l'air pur.

### Phase 2

Le patient, après avoir placé le masque 1 sur son visage, expire dans celui-ci, le mélange gazeux hypoxique provenant de ses poumons , c'est-à-dire un mélange composé d'oxygène, d'azote, de dioxyde de carbone, de vapeur d'eau et de diverses impuretés; sous l'effet du souffle, la valve 2 s'ouvre et permet au mélange gazeux de traverser le tuyau 3, le robinet 4, et le filtre 5, puis le mélange gazeux est pulsé par le ventilateur 6 à travers le robinet 7 vers l'électro-filtre 8 qu'il traverse avant d'arriver au niveau de la sonde 12 et de l'analyseur 13 qui indique au patient sa teneur en oxygène.

### Phase 3

Le patient aspire dans le masque 1 ce qui provoque l'ouverture de la valve 2' en permettant au mélange gazeux d'arriver dans le réservoir 15 après avoir passé dans le tuyau 3' et d'être inhalé par le patient.

En continuant de respirer dans le masque 1, le patient provoque la répétition périodique d'un cycle composé des phases 2 et 3 au cours duquel il aspire de façon continue un mélange gazeux hypoxique dont la teneur en oxygène peut être maintenue à une valeur relativement constante en observant les indications de l'analyseur 14 et en introduisant, si nécessaire une certaine quantité d'air pur dans le circuit au moyen des robinets 4 et 10.

Le filtre 5 a pour fonction de retenir le dioxyde de carbone, l'humidité et les impuretés contenues dans le mélange gazeux expiré par le patient. Il comprend un récipient hermétique rempli de granules constituées par de la poudre d'hydroxyde de fer agglomérée par un liant approprié. Ces granules ont un diamètre compris entre 1 à 5 mm ce qui leur confère une surface de contact maximum avec le flux gazeux.

Le ventilateur 6 a non seulement pour effet de pulsé le mélange gazeux mais encore de le rendre parfaitement homogène.

L'électro-filtre 8 a pour fonction d'éliminer, sous l'action de décharges électriques, les virus, les microorganismes et les aérosols finement divisés contenus dans le mélange expiré par le patient. Il est constitué par un boitier étanche en matériau diélectrique, dans lequel sont disposés en série et coaxialement, alternativement des électrodes 16 et des disques filtrants 17. Les électrodes 16 sont réalisées en au moins un métal du groupe d'éléments VIII ( nickel, cobalt, iron, acier, etc. ).

On applique d'abord un potentiel positif à l'une de ces électrodes puis un potentiel négatif à l'autre jusqu'à l'obtention d'une différence de potentiel comprise entre 2 et 9 kv.

Le mélange gazeux arrivant dans le réservoir 15 présente un degré de pureté satisfaisant les exigences les plus sévères en matière de traitements hypoxiques.

Lors de tels traitements, le patient respire par séquences, respectivement de l'air pur et le mélange gazeux hypoxique selon un programme déterminé par le médecin.

L'appareil représenté permet d'obtenir des mélanges hypoxiques dans lesquel l'oxygène se trouve en proportion de 8,5 à 20% en volume.

Les granules du filtre 5 pourront être régénérées en purgeant le circuit de l'appareil avec de l'air frais pendant les séquences au cours desquelles le patient respire de l'air pur.

L'appareil décrit sera avantageusement réalisé sous forme portative de manière à pouvoir être aisément transpoté d'un lieu à un autre.

Sa sou plesse d'utilisation et sa grande simplicité d'utilisation donne l'opportunité d'effectuer des séances de prophilaxie ou de thérapie hypoxique chez le patient.

Par ailleurs, il faut remarquer que son coût et ses frais d'exploitation sont très réduits par rapport à ceux des appareils connus.

## Revendications

1. Procédé pour produire un mélange gazeux hypoxique composé d'oxygène et d'azote dans les proportions respectives de 8 à 18% et 92 à 82% en volume, caractérisé par le fait qu'il consiste à appauvrir l'air en oxygène par la méthode de la respiration périodique et à éliminer le dioxyde de carbone et les impuretés contenues dans ce mélange.

2. Appareil pour effectuer des traitements hypoxiques mettant en oeuvre le procédé selon la revendication 1, caractérisé par le fait qu'il comprend un masque (1) destiné à être appliqué sur le visage du patient, au moins une valve (2,2'), et un ou plusieurs filtres (5,8) destinés à débarasser le mélange gazeux expiré par le patien du dioxyde de carbone et de ses impuretés.

3. Appareil selon la revendication 2, caractérisé par le fait que l'un des filtres (5) est constitué par un milieu absorbant comprenant des granules d'hydroxide de fer.

4. Appareil selon la revendication 2, caractérisé par le fait que l'un des filtres (8) est un électro-filtre.

5. Appareil selon la revendication 4, caractérisé par le fait que ledit électro-filtre (8) est constitué par un boitier en matériau diélectrique dans lequel sont disposés en série et coaxialement , alternativement des électrodes (16) et des disques filtrants (17).

6. Appareil selon la revendication 5, caractérisé par le fait que lesdites électrodes (16) sont réalisées en au moins un métal du groupe d'éléments VIII.

7. Appareil selon la revendication 2, caractérisé par le fait que ses éléments sont disposés dans un circuit fermé.

8. Appareil selon la revendication 7, caractérisé par le fait qu'il comprend un ventilateur (6) assurant la circuculation et l'homogénisation du mélange gazeux.

9. Appareil selon la revendication 7, caractérisé par le fait qu'il comprend des moyens (11,12) permettant de mesurer la proportion d'oxygène contenu dans le mélange

10. Appareil selon la revendication 9, caractérisé par le fait qu'il comprend des moyens (4,10) permettant de corriger la teneur en oxygène du mélange gazeux.
